# EUROPEAN PATENT APPLICATION

(11) **EP 4 530 601 A1**
(43) Date of publication of application: **02.04.2025**
(21) Application number: 23199861.8
(22) Date of filing: 26.09.2023
(51) Int. Cl.: G01N 1/12, G01N 1/14, G01N 1/20, G01N 33/18

(54) **PROCESS WATER SAMPLING IMMERSION PROBE**

(71) Applicant: Hach Lange GmbH, 14163 Berlin (DE)
(72) Inventor: WEBER, Roman, 14163 Berlin (DE); DRAEGER, Hartmut, 14163 Berlin (DE); STEINHAUER, Frank, 14163 Berlin (DE)
(74) Representative: terpatent PartGmbB

(57) **Abstract**

The invention refers to a process water sampling immersion probe (10) comprising a filter module (20) comprising a plane filter screen (22, 23) and a sample suction opening (28) through which filtered sample water is pumped from the filter module (20) to an analyzer unit (80), and a rigid holding structure (90) for holding the filter module (20),
wherein a swivel bearing arrangement (30) is provided, the swivel bearing arrangement (30) defining a static swivel axis (R) and connecting the filter module (20) with the rigid holding structure (90), so that the filter module (20) can swivel within a swivel sector (5) having a swivel angle of at least 10°.

## Description

The present invention relates to a process water sampling immersion probe for continuously filtering a water sample from water.

A process water sampling immersion probe is used as a part of a process water analysis arrangement for analyzing one or more analyte in water, for example in wastewater in a wastewater tank being a part of a wastewater treatment plant.

A stationary water sampling immersion probe arrangement is disclosed in US 6 379 621 B1. The arrangement comprises a streamlined filter module which is held in position within the wastewater tank by a stiff holding structure. The wastewater in the wastewater tank is flowing in a general flow direction but the flow direction of the wastewater can vary within an angle of +/- 30° or even more due to different influences so that the wastewater streams against the filter module from different angles. When the filter module is not orientated perfectly in-line with the wastewater current, clogging can appear at the lee side of the filter module.

It is an object of the invention to provide a process water immersion probe with improved self-cleaning qualities.

This object is solved with a process water sampling immersion probe with the features of main claim 1.

The process water sampling immersion probe for continuously filtering a water sample from water, preferably from wastewater, comprises a filter module with a filter screen, preferably with a substantially plane filter screen, more preferably with a plane filter screen standing in a vertical plane.

The filter module is provided with a suction opening through which filtered sample water is pumped from the filter module to a land-based analyzer unit. During operation, the filter module is immersed into the wastewater of a wastewater tank. A rigid and static holding structure fixed to the bank of the wastewater tank holds the filter module in position within the wastewater tank.

The process water sampling immersion probe is provided with a swivel bearing arrangement having a defined swivel axis and mechanically connecting the filter module with the stiff and rigid holding structure so that the filter module can swivel within a swivel sector around the swivel axis. The swivel sector of the swivel bearing arrangement has at least an angle of 10° so that the filter module can swivel with a swivel angle of +/-5° in relation to the center swivel position of the filter module. The center position of the swivelling filter module corresponds with the general flow direction of the wastewater current within the wastewater tank.

Since the filter module can swivel, the filter module can adapt its orientation to the local direction of the wastewater current so that the flow characteristics at the surface of the plane filter screen remain substantially constant. As a result, the plane filter screen generally is not in a lee position so that there is always a tangential laminar and/or a directly adjacent water flow at the surface of the filter screen which adjacent water flow avoids substantial clogging and growth of a clogging structure at the filter screen.

Since the filter module generally is in-line with the wastewater current, the holding forces for holding the filter module in position are relatively low so that the rigid holding structure can be configured less rigid.

Preferably, the defined swivel axis of the swivel bearing arrangement is arranged perfectly vertically.

Preferably, the filter module is fixed with its filter module end portion to the swivel bearing arrangement so that the filter module is hinged by the swivel bearing arrangement to the rigid holding structure as a flag to a flagpole. The complete filter module is always located at the downstream side of the swivel bearing arrangement.

Preferably, the swivel bearing arrangement is provided with at least two separate swivel bearing units which are, more preferably, provided at the two vertical end portions of the filter module. Preferably, the swivel bearing arrangement is provided with at least one bearing unit being of the friction bearing type. A friction bearing is mechanically simple and is more reliable in difficult environments than a rolling bearing.

Preferably, the swivel bearing arrangement is provided with a swivel limitation means limiting the possible swivel sector to a maximum swivel angle. Preferably, the swivel limitation means limits the swivel sector to a maximum total swivel angle of less than 91°, more preferably to a swivel angle of less than 75°. In a more preferred embodiment, the swivel angle is at least +/-15°, for a total swivel angle of at least 30°. In another preferred embodiment, the total swivel angle is between 30° and 75°.

The swivel limitation means makes a complete 360° rotation of the filter module in relation to the rigid holding structure impossible so that the fluidic and electric connections between the swivelling filter module and the rigid holding structure can be realized relatively simple and durable.

Preferably, at least one bearing shell of the friction bearing unit is made of plastic so that the frictional forces of the friction bearing unit are relatively low.

Preferably, the swivel bearing arrangement is provided with a wet bearing unit which is fluidically open to the environment and in particular to the wastewater into which the filter module is immersed. The wet bearing concept avoids all sealing measures which would be necessary for a dry bearing unit. However, the wet bearing unit needs to be configured and constructed relatively robust against water, debris and clogging etc.

Preferably, a flexible fluid hose is provided for fluidically connecting the sample suction opening of the filter module with a downstream fixed sample line being fixed to the rigid holding structure. Since the swivel bearing arrangement is provided with a swivel limitation means, the fluid connection between the swivelling filter module and a fixed fluid line at the rigid holding structure can be realized relatively simple and cost-effective.

Preferably, the swivel bearing arrangement is provided with at least one swivel bearing unit and is provided with a vertical protection shield surrounding the swivel bearing unit. The vertical protection shield is provided upstream of the swivel bearing unit so that the swivel bearing unit is protected against any direct impacts of corpuscular components of the wastewater current.

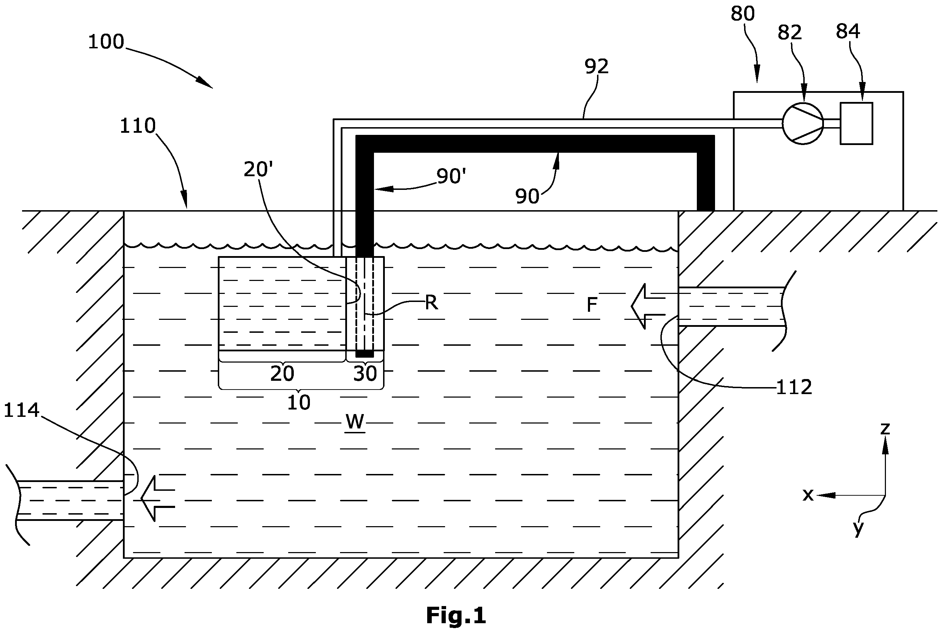

One embodiment of the invention is described below with reference to the enclosed drawings, wherein figure 1 schematically shows a process water analysis arrangement comprising a process water sampling immersion probe immersed into the wastewater of a wastewater tank,

figure 2 shows a vertical cross-section II-II of the process water sampling immersion probe of figure 1, and

figure 3 shows a horizontal cross-section III-III of the process water sampling immersion probe of figures 1 and 2.

Figure 1 schematically shows a process water analysis arrangement 100 continuously analyzing water samples from wastewater W. The wastewater W continuously flows through a tank inlet 112 into a wastewater tank 110 and continuously flows out of the wastewater tank 110 through a tank outlet 114 so that a substantially continuous wastewater flow F with a substantially constant flow direction is caused. The wastewater tank 110 is part of a wastewater treatment plant (not shown). The process water analysis arrangement 100 is provided for quasi-continuously determining the concentration of one or more analyte of the wastewater W, for example of ammonium and/or phosphate.

The process water analysis arrangement 100 comprises a process water sampling immersion probe 10 which is completely immersed into the wastewater W and which is held in position by a stiff and rigid holding structure 90 which is mounted to the border of the wastewater tank 110. The immersion probe 10 is fluidically and electronically connected to a land-based analyzer unit 80 comprising an electric sample pump 82 and an analyzer device 84 for analyzing the concentration of one or more analyte of the filtered water sample of the wastewater W.

The immersion probe 10 comprises a flat, plane and rectangular filter module 20 lying in a vertical plane xz and having two parallel vertical filter screens 22, 23 defining a filter module cavity 25 therebetween. The filter module 20 comprises a horizontal top filter module wall 29 with a sample suction opening 28 through which the filtered sample water is pumped by the sample pump 82 from the filter module 20 via a flexible fluid hose 92' and a downstream fixed sample line 92 to the analyzer device 84 of the analyzer unit 80. The filter module 20 is not provided with any mechanic cleaning means, and in particular is not provided with any air bubble generating means generating an air bubble curtain.

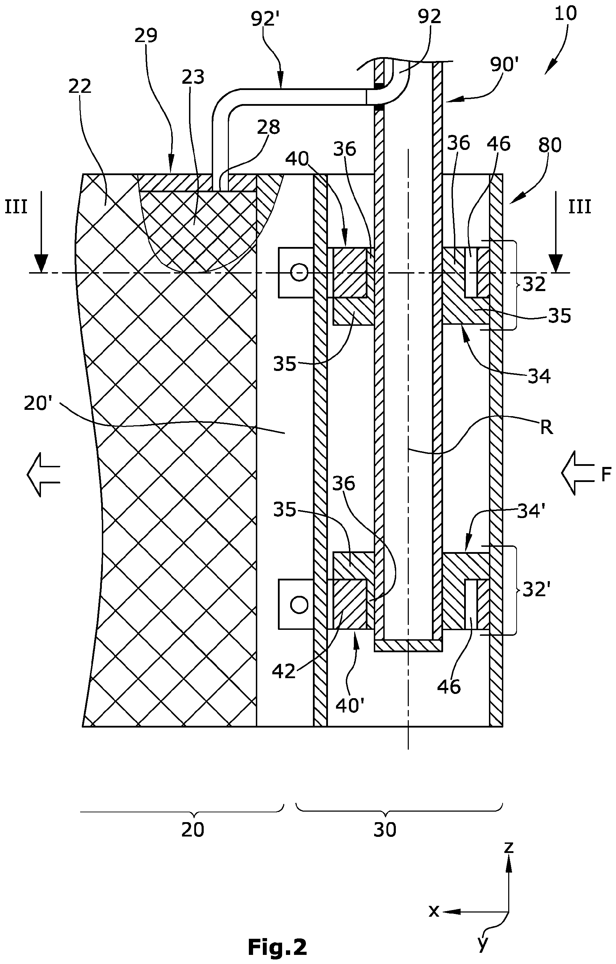

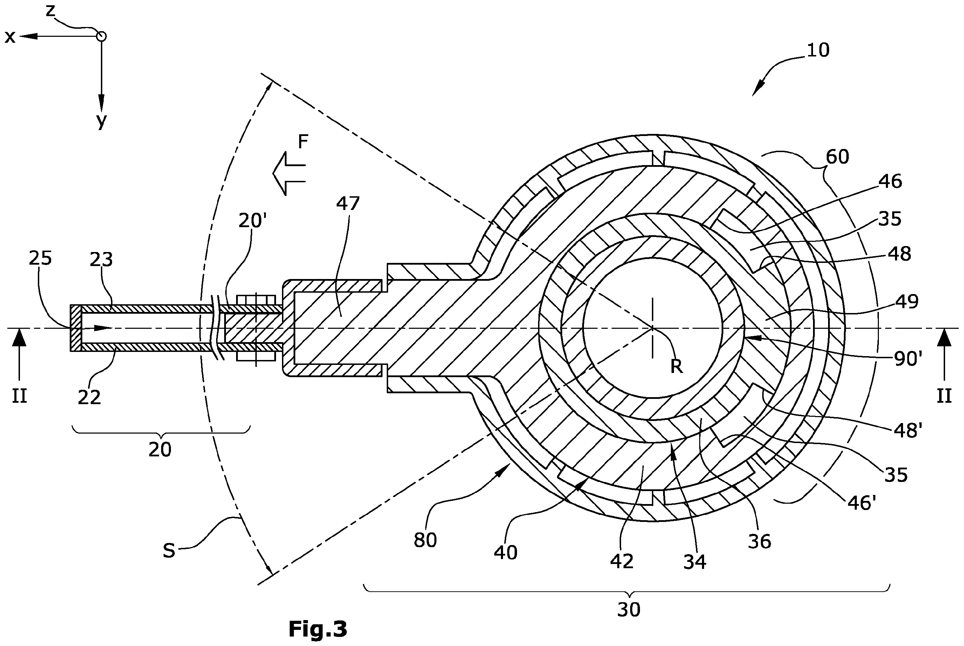

The process water sampling immersion probe 10 is provided with a swivel bearing arrangement 30 defining a vertical swivel axis R, which swivel bearing arrangement 30 connects the swivelling filter module 20 with a vertical hollow bearing axis tube 90' held by the rigid holding structure 90 in position. The swivel bearing arrangement 30 comprises two separate wet and frictional swivel bearing units 32, 32'. Each swivel bearing unit 32, 32' comprises an inner bearing shell 34, 34' made of a plastic body defining a radial bearing cylinder 36 and defining an axial bearing ring body 35, and comprises an outer bearing shell 40, 40' as shown in figures 2 and 3. The cylindrical outer bearing shell 40, 40' is defined by a complex metal or plastic body 42 which also defines a radial fixation nose 47. The fixation nose 47 is fixed to a filter module end portion 20' of the filter module 20, so that the filter module 20 is hinged to the bearing axis tube 90' as a flag to a flag post.

As shown in figure 3, the swivel bearing units 32, 32' both also are provided with a swivel limitation means 60 limiting the swivel sector S to a maximum total swivel angle of 60°. The swivel limitation means 60 is defined by two tangential swivel stops 46, 46' of each outer bearing shell 40, 40' and by two cooperating tangential swivel stops 48, 48' defined by a stop nose 49 of the corresponding inner bearing shell 34, 34'.

The swivel bearing units 32, 32' are shielded by a cylindrical vertical protection shield 80 surrounding the swivel bearing units 32, 32' so that the swivel bearing units 32, 32' are protected against direct impacts of corpuscular components of the wastewater current.

In the figures, the vertical direction is indicated by z, the horizontal longitudinal direction of the water flow F is indicated by x, and the transversal horizontal direction is indicated by y.

## Claims

1. A process water sampling immersion probe (10) comprising a filter module (20) comprising a plane filter screen (22, 23) and a sample suction opening (28) through which filtered sample water is pumped from the filter module (20) to an analyzer unit (80), and
a rigid holding structure (90) for holding the filter module (20), wherein a swivel bearing arrangement (30) is provided, the swivel bearing arrangement (30) defining a static swivel axis (R) and connecting the filter module (20) with the rigid holding structure (90), so that the filter module (20) can swivel within a swivel sector (S) having a swivel angle of at least 10°.

2. The process water sampling immersion probe (10) of claim 1, wherein the defined swivel axis (R) of the swivel bearing arrangement (30) is arranged vertically.

3. The process water sampling immersion probe (10) of one of the preceding claims, wherein the filter module (20) is fixed with a filter module end portion (20') to the swivel bearing arrangement (30).

4. The process water sampling immersion probe (10) of one of the preceding claims, wherein the swivel bearing arrangement (30) is provided with at least two separate swivel bearing units (32, 32').

5. The process water sampling immersion probe (10) of one of the preceding claims, wherein the swivel bearing arrangement (30) is provided with at least one bearing unit (32, 32') being of the friction bearing type.

6. The process water sampling immersion probe (10) of one of the preceding claims, wherein the swivel bearing arrangement (30) is provided with a swivel limitation means (60) limiting the possible swivel sector (S) to a maximum swivel angle.

7. The process water sampling immersion probe (10) of claim 6, wherein the swivel limitation means (60) limits the swivel sector (S) to a maximum swivel angle of less than 91°, preferably of less than 75°.

8. The process water sampling immersion probe (10) of one of the preceding claims 5 to 7, wherein at least one bearing shell (34) of the bearing unit (32, 32') is made of plastic.

9. The process water sampling immersion probe (10) of one of the preceding claims, wherein the swivel bearing arrangement (30) is provided with a wet bearing unit (32, 32') which is fluidically open to the environment.

10. The process water sampling immersion probe (10) of one of the preceding claims, wherein a flexible fluid hose (92') is provided fluidically connecting the sample suction opening (28) of the filter module (20) with a downstream fixed sample line (92) being fixed to the rigid holding structure (90).

11. The process water sampling immersion probe (10) of one of the preceding claims, wherein the swivel bearing arrangement (30) is provided with at least one swivel bearing unit (32, 32') and with a vertical protection shield (80) surrounding the swivel bearing unit (32, 32').

## Amended claims

### Amended claims in accordance with Rule 137(2) EPC.

1. A process water sampling immersion probe (10) being immersed into wastewater (W) of a wastewater tank (110), comprising a filter module (20) comprising a vertical plane filter screen (22, 23) and a sample suction opening (28) through which filtered sample water is pumped from the filter module (20) to a land-based analyzer unit (80), and
a rigid and static holding structure (90) for holding the filter module (20),
wherein a swivel bearing arrangement (30) is provided, the swivel bearing arrangement (30) defining a static swivel axis (R) and connecting the filter module (20) with the rigid holding structure (90), and
wherein the defined swivel axis (R) of the swivel bearing arrangement (30) is arranged vertically,
so that the filter module (20) can swivel within a swivel sector (S) having a swivel angle of at least 10°, so that the filter module (20) can adapt its orientation to the wastewater current.

2. The process water sampling immersion probe (10) of claim 1, wherein the filter module (20) is fixed with a filter module end portion (20') to the swivel bearing arrangement (30).

3. The process water sampling immersion probe (10) of one of the preceding claims, wherein the swivel bearing arrangement (30) is provided with at least two separate swivel bearing units (32, 32').

4. The process water sampling immersion probe (10) of one of the preceding claims, wherein the swivel bearing arrangement (30) is provided with at least one bearing unit (32, 32') being of the friction bearing type.

5. The process water sampling immersion probe (10) of one of the preceding claims, wherein the swivel bearing arrangement (30) is provided with a swivel limitation means (60) limiting the possible swivel sector (S) to a maximum swivel angle.

6. The process water sampling immersion probe (10) of claim 5, wherein the swivel limitation means (60) limits the swivel sector (S) to a maximum swivel angle of less than 91°, preferably of less than 75°.

7. The process water sampling immersion probe (10) of one of the preceding claims 4 to 6, wherein at least one bearing shell (34) of the bearing unit (32, 32') is made of plastic.

8. The process water sampling immersion probe (10) of one of the preceding claims, wherein the swivel bearing arrangement (30) is provided with a wet bearing unit (32, 32') which is fluidically open to the environment.

9. The process water sampling immersion probe (10) of one of the preceding claims, wherein a flexible fluid hose (92') is provided fluidically connecting the sample suction opening (28) of the filter module (20) with a downstream fixed sample line (92) being fixed to the rigid holding structure (90).

10. The process water sampling immersion probe (10) of one of the preceding claims, wherein the swivel bearing arrangement (30) is provided with at least one swivel bearing unit (32, 32') and with a vertical protection shield (99) surrounding the swivel bearing unit (32, 32').
